# EUROPEAN PATENT SPECIFICATION

(11) **EP 3 716 929 B1**
(45) Date of publication and mention of the grant of the patent: **19.01.2022**
(21) Application number: 17808405.9
(22) Date of filing: 27.11.2017
(51) Int. Cl.: A61F 13/551, A61F 13/496

(54) **PANT-TYPE ABSORBENT ARTICLE WITH A DISPOSAL TAPE**
HOSENARTIGER SAUGFÄHIGER ARTIKEL MIT EINEM ENTSORGUNGSBAND
ARTICLE ABSORBANT DE TYPE CULOTTE AVEC BANDE JETABLE

(43) Date of publication of application: 07.10.2020
(73) Proprietor: Essity Hygiene and Health Aktiebolag, 405 03 Göteborg (SE)
(72) Inventor: VAN SCHIE, Bert, 405 03 Göteborg (SE); GRAHN, Louise, 405 03 Göteborg (SE); JOHANSSON, Marie, 405 03 Göteborg (SE)
(74) Representative: Hoffmann Eitle
(86) International application number: PCT/EP2017/080539
(87) International publication number: WO 2019/101346

(56) References cited:
- WO-A1-97/16146
- WO-A1-99/17693
- US-A- 5 626 573
- US-A1- 2015 080 829
- US-A1- 2017 281 430

## Description

### TECHNICAL FIELD

The present disclosure pertains to a pant-type absorbent article with a disposal tape. The present disclosure also pertains to a method of permanently attaching a disposal tape to the outer cover of a pant-type absorbent article.

### BACKGROUND

Disposable absorbent articles are designed to receive and contain body exudates or fluids such as urine. The absorbent articles are configured for single use and comprise a liquid permeable topsheet, an outer cover and an absorbent core arranged between the topsheet and the outer cover.

The disposable absorbent articles should be comfortable during use, while being worn, and it may be desired that the absorbent articles have cloth-like and soft appearance as close to regular underwear as possible. For disposable pant-type diapers, the outer cover may often be folded inwardly over the waist edge and is therefore in direct contact with the skin of the wearer and it may therefore be of an advantage to have a soft and cloth-like nonwoven as the outermost material layer on the outer cover.

Disposable pants-type diapers may be provided with various types of disposal tape arrangements to secure a rolled-up garment, and thereby enable easy and hygienic disposal of the diaper. Such disposal tape may be a longitudinally extending fastening tape having an adhesive section releasably bonded to the outer cover of the diaper. The upper end or lower end of the tape is permanently bonded to the outer surface of the garment. The disposal tape can be stretched after peeling a portion of the tape from the base, and then wrapped around the diaper to secure the diaper in a rolled-up configuration for easy disposal. During use of the diaper, rubbing and chafing against clothes of the user may result in detachment of the disposal tape edges, or corner portions, if the attachment of the disposal tape to the outer cover is not sufficient, which gives a negative impression on the quality of the diaper upon disposal, partial detachment of the disposal tape from the outer cover may occur upon unfolding and pulling in the disposal tape when wearer or care giver are wrapping the disposal tape around the rolled up diaper. US 2017/281430 A1, US 2015/080829 A1, WO 99/17693 A1 and WO 97/16146 A1 disclose each such kind of tapes.

### SUMMARY

The present disclosure offers an improved permanent attachment of the disposal tape to the outer cover of pant-type absorbent articles and a method for permanently attaching the disposal tape to the outer cover of pant-type absorbent articles.

The invention concerns a pant-type diaper according to claim 1 and a method according to claim 16. Further embodiments are set out in the dependent claims, in the following description and in the drawings.

The present disclosure relates to a pant-type absorbent article such as a pant diaper, a sanitary pant or incontinence pant. The article comprises an outer cover being disposed on a garment facing side of the absorbent article and the article further has a front portion, a back portion and a crotch portion therebetween. The absorbent article furthermore comprises an absorbent core arranged in the crotch portion on a wearer facing side of the outer cover. The outer cover comprises a nonwoven fibrous material on a garment-facing side thereof and a disposal tape comprising a tape adhesive zone is permanently attached at the tape adhesive zone to the outer cover in an attachment zone on the nonwoven fibrous material of the outer cover in the back portion of the article. The disposal tape has a folded pre-disposal configuration and an unfolded disposal configuration. The outer cover attachment zone is constituted by a modified portion of the nonwoven fibrous material, wherein a bond strength between the tape adhesive zone and the modified attachment zone is greater than a bond strength between the tape adhesive zone and a non-modified portion of the nonwoven fibrous material.

The term "absorbent article" refers to products that are placed against the skin of the wearer to absorb and contain body exudates, like urine, faeces and menstrual fluid. The disclosure furthermore refers to disposable absorbent articles, which means articles that are not intended to be laundered or otherwise restored or reused as an absorbent article after use. The disclosure refers to "disposable pant-type absorbent articles", having an outer cover being disposed on a garment facing side of the absorbent article and an absorbent core arranged in the crotch portion on a wearer facing side of the outer cover.

Examples of such pant-type absorbent articles are pant diapers, sanitary pants and incontinence pants.

By "permanently attached" refers to the attachment of two elements, herein a nonwoven fibrous material to a disposal tape, such that the elements tend to be and remain bonded during normal use conditions of the absorbent article, such as for example that the disposal tape is permanently attached to the nonwoven fibrous material with such adhesion that the tape withstands a load of at least 25 N in a gripping end of the disposal tape without the tape coming away from the nonwoven fibrous material.

The pant-type absorbent article comprises an outer cover, the outer cover may be elastic, and may comprise an elasticized waist portion, such as comprising elastic members, such as elastic threads, affixed between the outer cover garment facing nonwoven material and a further material layer provided on a wearer facing side of the nonwoven material. The outer cover may also comprise an elastic laminate, such as for example elastic laminate comprising an elastic film which on opposite sides is bonded to first and second non-elastic fibrous nonwoven material. The laminate is made by bonding the non-elastic fibrous nonwoven materials to the elastic film layer and subsequently stretching the composite material. The elastic film material may be of a breathable material.

The modified attachment zone may be a heat-treated zone, such as a heat-treated zone formed by means of heat embossing, ultra sound, heated air or IR. The heat treatment of the nonwoven fibers results in that the polymeric material in the nonwoven fibers softens and/or melts and thereby modifies the nonwoven surface.

The modified attachment zone may be a compressed area, such as a compressed zone formed by means of embossing, such as by a stamp or by a roller. The compression of the nonwoven fibrous material compresses and flattens the nonwoven material which provides a bonding surface.

The modified attachment zone may have a modified cross-section appearance, such that the nonwoven fibers are at least partly consolidated or flattened within the attachment zone. Such modification may be formed by heat-treating and/or compressing and modifying the attachment zone such that the bond strength to the disposal tape adhesive zone is improved. A combination of heat and compression may be achieved using heated rollers and heat embossing, which softens and/or melts the polymeric material in the nonwoven while also providing pressure to provide a flattened surface.

The modified attachment zone may be a plasma-treated zone, such as atmospheric pressure plasma, or a corona-treated zone. Plasma and corona treatments increase the bond strength between the nonwoven outer cover and the disposal tape by modifying the surface of the nonwoven.

A plasma treatment modifies the nonwoven fiber surface on a nano-scale by changing the chemical structure of the fiber. Plasma is an outwards neutral, partially ionized gas, the composition of which depends on the gas used in the formation of plasma. Gas molecules are ionized in an electric field through electron impacts, and the ionized, highly reactive species, such as ions, electrons and radicals, modify the surface of the nonwoven material. Plasma treatments provide a permanent surface modification.

Corona treatments use a low corona discharge to impart changes in the properties of the nonwoven surface. The surface modification provided by the corona treatment is not permanent.

The outer cover may be a laminate of the nonwoven fibrous material and at least one further material layer, such as a nonwoven material and/or a thermoplastic film.

The laminate may have a lamination bonding area of from 5 to 100 %. The laminate may be bonded be means of adhesive slot bonding or spray coating. Optionally, the laminate bonding area may be below 100%, such as from 5% to 95%.

The nonwoven fibrous material may comprise a plurality of discrete bonds, such as thermal bonds, and the cumulated surface area of the discrete bonds may be in the range of from 1% to 25% of the total surface area of the nonwoven fibrous material, such as from 5% to 25% of the total surface area of the nonwoven fibrous material. It has been found that nonwoven materials having a low cumulated area of discrete bonds provide a soft and underwear-like appearance to the nonwoven material. The discrete bonds may further have a distance to adjacent discrete bonds of at least 2 mm, or at least 4 mm, the distance being measured from edge to edge of the discrete bonds. The nonwoven fibrous material may furthermore be treated with a melt additive to increase the softness of the nonwoven material, examples of such melt additives are polyolefin waxes and primary and secondary amides, such as erucamide, oleamide and stearyl derivatives. However, the fact that the nonwoven material is soft and fluffy renders it more difficult to attach the disposal tape to the nonwoven fibrous material. A problem also noticed by the present inventors in connection with absorbent articles having softer nonwoven fibrous materials as outer covers, is that the bond strength between a softer nonwoven material and a disposal tape is not strong enough, which may lead to partial detachment of the disposal tape when using it for wrapping the used absorbent article.

The nonwoven fibrous material may thus be a soft nonwoven fibrous material, for example a spunbond nonwoven material, a laminate of a spunbond-meltblown-spunbond material. The nonwoven fibrous material may be a carded nonwoven material and the nonwoven fibrous material may be a through-air-bonded nonwoven material. To increases the softness of the nonwoven fibrous material, the nonwoven materials may have a low cumulative area of thermal discrete bonds.

The nonwoven fibrous material may have a higher bond area in the modified attachment zone than in the non-modified portion of the nonwoven fibrous material.

The cumulated surface area of the bonds in the modified attachment zone may be at least 30%, such as from 30% to 85%, of the total surface area of the modified attachment zone.

For an absorbent article comprising an elastic outer cover, there may also exist a need for a strong attachment between the outer cover nonwoven material and the disposal tape as the wrinkles resulting from the elastic outer cover may result in weaker bonding of the disposal tape and/or increased risk of the disposal tape outer edges to detach from the outer cover.

The disposal tape may have an outer circumferential edge in the folded pre-disposal configuration and the outer cover attachment zone may have an outer circumferential edge. The outer cover attachment zone may extend to or beyond the outer circumferential edge of the disposal tape when arranged in the unfolded pre-disposal configuration. The outer cover attachment zone may extend beyond the outer circumferential edge of the disposal tape by at least 1 mm, or at least 2 mm, or at least 5 mm.

The fact that the outer cover attachment zone may extend to or beyond the outer circumferential edge of the disposal tape provides higher bond strength between the nonwoven fibrous material and the disposal tape at the circumferential edge of the disposal tape which prevents the outer edges to loosen from the nonwoven fibrous material which may cause the disposal tape to get caught in the wearer clothing and result in premature unfolding of the disposal tape.

The attachment zone may be modified in an intermittent pattern or in a continuous pattern covering entirely the outer cover attachment zone.

The disposal tape may be rectangular and may be z-folded when in the pre-disposal configuration.

The disposal tape comprises or consists of a thermoplastic film.

The disposal tape may be permanently attached to the attachment zone by means of an adhesive. The adhesive may for example be a pressure-sensitive hot melt (PSHM) adhesive.

The disposal tape may have a length and a width in the unfolded configuration and, the width may be in the range from 5 to 30 mm, such as from 12 to 20 mm. It has been discovered by the inventors that a disposal tape having width of at least 5 mm, such as at least 12 mm, and being adhered adhesively to an outer cover nonwoven fibrous material being modified according to the present disclosure gives a significantly improved attachment of the disposal tape.

The present disclosure also pertains to a method of permanently attaching a disposal tape to an outer cover of a pant-type article, comprising:
a) modifying a zone of a nonwoven fibrous material with heat treatment, plasma treatment, by compressing the zone or a combination of two or more of the treatments to provide a modified attachment zone;
b) permanently attaching a disposal tape comprising a tape adhesive zone to the nonwoven fibrous material by adhesively bonding the tape adhesive zone to the modified attachment zone;
c) producing a pant-type absorbent article comprising the nonwoven fibrous material as an outer cover being disposed on a garment facing side of the absorbent article, the article having a front portion, a back portion and a crotch portion therebetween and comprises an absorbent core arranged in the crotch portion,
wherein the zone modification step a) and the disposal tape attachment step b) are carried out prior to, during or after the pant-type absorbent article assembly step c).

The attachment zone may be modified by one or more of the following treatments; a heat or cold embossing, ultra sound, plasma charges, such as corona treatment, heated air and/or infrared radiation.

The step c) of producing an absorbent article may include elastifying the outer cover by means of elastic elongated elements or an elastic material or wherein the wherein the nonwoven fibrous material is an elastic nonwoven fibrous material, providing an at least partly elastic outer cover and wherein the elastic outer cover is stretched during the zone modification step a) and the disposal tape attachment step b).

The modification step a) may comprise a step of simultaneous heat and compression treatment, such as by heat embossing, for example by a heated roller or a heated stamp.

The fact that the modification step may be carried out with a simultaneous heat and compression treatment provides an improved bond strength between the tape adhesive zone and the modified attachment zone without the need to use too high temperatures or too high pressures, which could otherwise risk to impair the nonwoven fibrous material or require heating of the compression tool to high temperatures resulting in high energy consumption in order to obtain a desired modified nonwoven fibrous structure. Such modification may result in that the nonwoven fibers in the modified attachment zone are at least partly consolidated and flattened.

The modification step a) may comprise modifying a zone on the nonwoven fibrous material with heat treatment, and step b) is performed immediately after step a) while the modified attachment zone still has a temperature above the temperature of a non-modified portion of the nonwoven fibrous material.

### BRIEF DESCRIPTION OF THE DRAWINGS

The present disclosure will be further explained hereinafter by means of non-limiting examples and with reference to the appended drawings wherein:
- Fig. 1: illustrates a pant-type absorbent article with a disposal tape attached to the article in a folded pre-disposal configuration;
- Fig. 2: illustrates a pant-type absorbent article prior to attachment of the disposal tape;
- Fig. 3: illustrates a pant-type absorbent article with the disposal tape attached to the article in an unfolded disposal configuration;
- Fig. 4a: illustrates a disposal tape attached to a prior art non-modified nonwoven fibrous material;
- Fig. 4b: illustrates a disposal tape attached to a heat modified portion nonwoven fibrous material; and
- Fig. 4c: illustrates a disposal tape attached to a plasma modified nonwoven fibrous material.

### DETAILED DESCRIPTION

It is to be understood that the drawings are schematic and that individual components, such as layers of materials are not necessarily drawn to scale. The pant-type absorbent article shown in the figures are provided as examples only and should not be considered limiting to the disclosure. Accordingly, the scope of the invention is determined solely by the scope of the appended claims.

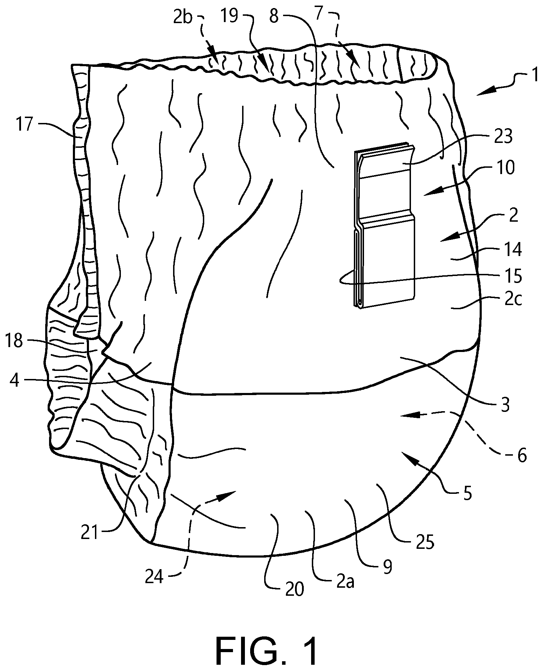

Fig. 1 is a perspective view of the pant-type absorbent article 1 according to the present disclosure. The pant diaper 1 has an outer cover 2, a topsheet 24 and an absorbent core 25 arranged between the topsheet 24 and the outer cover 2. The pant diaper 1 has a front portion 7, a back portion 8 and a crotch portion 9 and seams 17 joining the front portion 7 and the back portion 8 to form two leg openings 18 and a waist opening 19. The outer cover 2 illustrated in Fig. 1 is in the form of a multicomponent outer cover 2, comprising an outer cover main portion 2a including breathable outer cover front portion 2b and breathable outer cover back portion 2c. The outer cover main portion 2a is joined to the topsheet 24 around the periphery of the absorbent core 25, so that the absorbent core 25 is enclosed within the envelope formed by the topsheet 24 and the outer cover main portion 2a. The outer cover main portion 2a may be breathable to allow vapor to escape from the absorbent core 25 while still preventing liquids to pass therethrough.

The outer cover 2 may however also be formed of a continuous outer nonwoven fibrous material 4 extending over the outer cover main portion 2a, the front portion 2b and the back portion 2c. The outer cover 2 may be reinforced in the outer cover main portion 2a extending over the crotch area of the wearer, with a breathable or non-breathable polymeric film or with a film/nonwoven laminates.

Examples of breathable materials are microporous polymeric films, nonwoven laminates from spunbond and meltblown layers, laminates from microporous polymeric films and nonwoven materials. In the absorbent core 25 area, covering the absorbent core, outer cover 2 may comprise a liquid impervious film layer, such as a thin thermoplastic film, e.g. a polyethylene or polypropylene film. The liquid impervious film layer covers at least the absorbent core 25 on the garment-facing side of the pant diaper 1.

The liquid permeable topsheet 24 can be any suitable topsheet material as known by the person skilled in the art and may be fibrous topsheet material composed of a nonwoven material, e.g. spunbonded, meltblown, carded, hydroentangled, wet laid etc. Suitable nonwoven materials can be composed of natural fibers, such as woodpulp or cotton fibres, synthetic thermoplastic fibres, such as polyolefins, polyesters, polyamides and blends and combinations thereof or from a mixture of natural and synthetic fibres. Further examples of topsheet materials are porous foams. The materials suited as topsheet materials should be soft and non-irritating to the skin and be readily penetrated by body fluid, such as urine or menstrual fluid. The topsheet material may be essentially constituted of non-absorbent fibers, such as synthetic thermoplastic fibers, such as such as polyolefins, polyesters, polyamides and blends and combinations thereof. The synthetic fibers may be mono component fibers, bicomponent fibers or multicomponent fibers including polyesters, polyamides and/or polyolefins such as polypropylene and polyethylene.

The disposable absorbent article in this disclosure includes an absorbent core 25, provided on a wearer-facing side of the outer cover. The absorbent core 25 may be of any conventional kind and of any useful size and shape. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent structure. It is also common to have absorbent structures including layers of different material with different properties with respect to liquid acquisition capacity, liquid distribution capacity and storage capacity. This is well-known to the person skilled in the art and does therefore not have to be described in detail. The thin absorbent bodies, which are common in today's sanitary articles, often include a compressed mixed or layered structure of cellulosic fluff pulp and superabsorbent. The size and absorbent capacity of the absorbent structure may be varied to be suited for different uses such as, adult incontinence pants and baby pant diapers.

In Fig. 1, the outer cover 2 is made of another web material in the outer cover main portion 2a, which material provides comfort and breathability. The outer cover front portion 2b and the outer cover back portion 2c each comprises a soft nonwoven fibrous material 4 on the garment facing side 3 thereof.

The pant diaper 1 in Fig. 1 furthermore comprises barrier leg cuffs 21 disposed along longitudinal side edges of the pant diaper 1 in the crotch portion 9. The barrier leg cuffs 21 provide improved containment of liquids and other body exudates in the crotch portion 9.

The nonwoven fibrous material 4 may be a soft nonwoven fibrous material 4, for example a spunbond nonwoven material, a laminate of a spunbond-meltblown-spunbond material. The nonwoven fibrous material may be a carded nonwoven material and the nonwoven fibrous material may be a through-air-bonded nonwoven material. Nonwoven fibrous materials having a low cumulated area of discrete thermal bonds have been found to have improved softness. The discrete thermal bonds may be located in a sparse pattern including discrete bonds having a distance to adjacent bonds of at least 2 mm, or at least 4 mm, the distance being measured between edge to edge of the discrete bonds. The cumulated surface area of the bonds may be in the range of from 1% to 25% of the total surface area of the nonwoven material 4. The nonwoven material 4 may furthermore be treated with a melt additive to increase the softness of the nonwoven material 4.

A disposal tape 10 is attached to the nonwoven fibrous material 4 of the outer cover 2 in the back portion 8 of the pant diaper 1. The disposal tape 10 is attached to the pant diaper 1 with an adhesive, in a folded pre-disposal configuration. The adhesive may for example be a pressure sensitive hot melt (PSHM) adhesive. The disposal tape 10 may for example comprise or consist of a thermoplastic film, such as a polyolefin film. The disposal tape 10 has an outer circumferential edge 15 in the folded pre-disposal configuration.

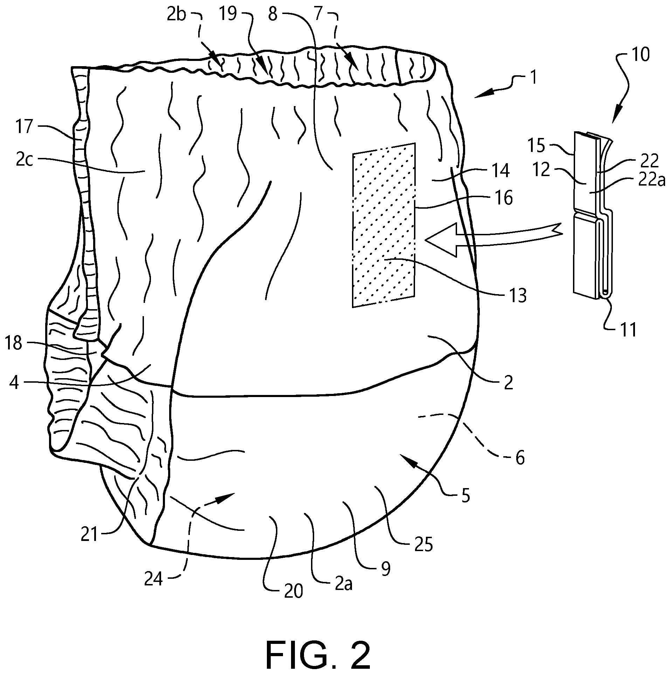

Fig. 2 illustrates a view of the pant-type diaper 1 according to Fig. 1 prior to attachment of the disposal tape 10 to the outer cover 2 in the back portion 8 of the absorbent article 1. The disposal tape 10 comprises a tape adhesive zone 12 provided with an adhesive for permanently attaching the disposal tape 10 to the outer cover 2 in a modified attachment zone 13 on the nonwoven fibrous material 4. The modified attachment zone 13 is constituted of a modified part of the nonwoven fibrous material 4 which has been modified to increase the bond strength between the tape adhesive zone 12 and the modified attachment zone 13. The modification of the nonwoven fibrous material 4 in the modified attachment zone 13 thus provides higher bond strength between the tape adhesive zone 12 and the modified attachment zone 13 in comparison to a non-modified portion 14 of the nonwoven fibrous material 14.

The bond strength may be measured with any method relevant for measuring the bond strength between the tape adhesive zone 12 and the modified attachment zone 13 and comparing it with bond strength between the tape adhesive zone 12 and the non-modified portion 14 of the nonwoven fibrous material.

Fig. 2 illustrates a modified attachment zone 13 having a modified cross-section appearance, wherein the nonwoven fibers are at least partly consolidated and/or flattened within the attachment zone 13.

The modification of the nonwoven fibrous material 4 and the attachment of the disposal tape 10 to the nonwoven fibrous material 4 may optionally be performed prior to assembly of the pant diaper 1. The nonwoven fibrous material 4 may be provided in the form of a continuous nonwoven fibrous material web and by modifying zones on the continuous nonwoven fibrous material web with a predefined distance between the zones. The modification may be achieved, as set out herein, by means of heat treatment, plasma treatment, by compressing said zone or a combination of two or more of said treatments to provide the modified attachment zone 13. In a subsequent step a disposal tape 10 may be attached to each modified attachment zone 13. The disposal tape 13 is attached at the tape adhesive zone 12, provided with an adhesive layer, to the nonwoven fibrous material 4 by pressing and thereby bonding the tape adhesive zone 12 to the modified attachment zone 13. The modification of the nonwoven and the attachment of the disposal tapes may be performed either in a separate production step, such that the nonwoven material may be delivered to the pant-type diaper assembly line in rolls with the disposal tapes already attached to the modified attachment zones at predefined and repeated locations, or alternatively when producing pant-type absorbent articles on a continuous production line, prior to, during or after the assembly of the pant-type absorbent article.

As illustrated in Fig. 2, the outer cover attachment zone 13 has an outer circumferential edge 16, and the outer cover attachment zone 13 extends to the outer circumferential edge 15 of the disposal tape 10 when attached to the outer cover 2 and arranged in the unfolded pre-disposal configuration. The outer circumferential edge 16 of the outer cover attachment zone 13 may also extend beyond the outer circumferential edge 15 of the disposal tape 10, such as by at least 1 mm, or at least 2 mm.

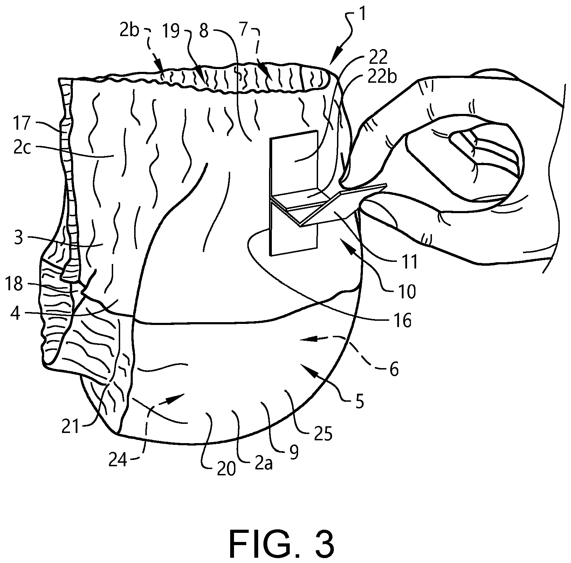

Fig. 3 illustrates a pant diaper 1 according to the present disclosure with the disposal tape 10 in an unfolded disposal configuration. The disposal tape 10 will unfold upon pulling in the gripping end 23 of the disposal tape 10.

The disposal tape 10 may comprise a first tape 22 and a second tape 11 attached to the outer cover 2, the first tape 22 comprising a first tape reinforcing section 22a and a first tape connection section 22b being attached to the second tape 10, such disposal tape configuration having an T-shaped attachment configuration when the disposal tape 10 is in an unfolded disposal configuration provides an improved attachment to the nonwoven fibrous material 4.

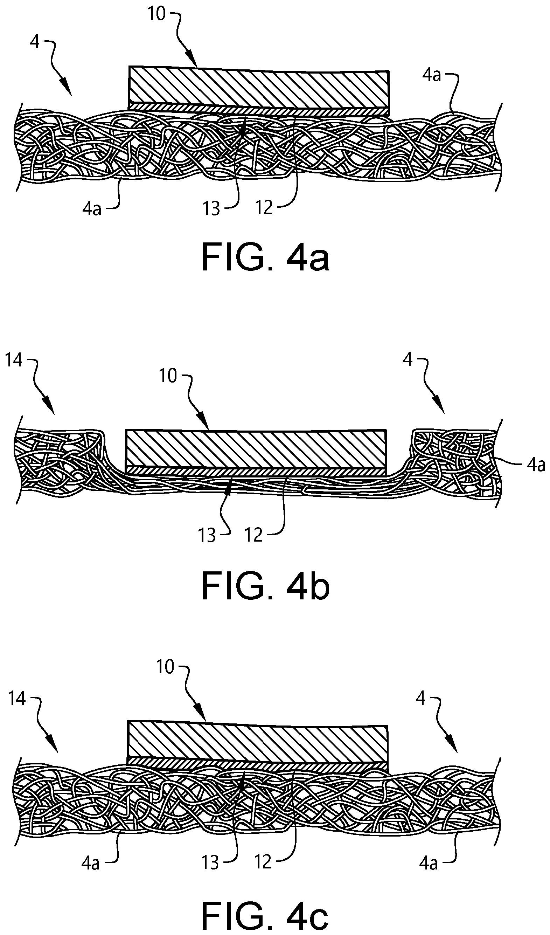

Fig. 4a illustrates a soft and non-modified prior art nonwoven fibrous material 4 comprising nonwoven fibers 4a, wherein a disposal tape 10 has been attached to the non-modified nonwoven fibrous material 4 in a non-modified attachment zone 13. The disposal tape 10 has a tape adhesive zone 12 provided with an adhesive layer. As may be seen in Fig. 4a, the hydrophobic nature of the nonwoven fibers 4a and the fact that the nonwoven fibrous material 4 has a relatively low total bonding area, i.e. cumulated surface area of the discrete bonds, making the material softer, also leads to a reduced contact area between the nonwoven material 4 and the disposal tape 10. This results in less strong bond strength between the tape adhesive zone 12 and the attachment zone 13.

Fig. 4b illustrates a nonwoven fibrous material 4 comprising nonwoven fibers 4a, wherein the nonwoven fibrous material 4 has been modified by heat embossing, such that the nonwoven fibers 4a are at least partly consolidated and flattened within an attachment zone 13. A disposal tape 10 is attached at a tape adhesive zone 12 to the nonwoven fibrous material 4 in the attachment zone 13. The fact that the attachment zone 13 is heat-embossed increases the bond strength between the tape adhesive zone 12 and the heat-embossed attachment zone 13.

Fig. 4c illustrates a nonwoven fibrous material 4 comprising nonwoven fibers 4a, wherein the nonwoven fibrous material 4 has been modified by plasma treatment in an attachment zone 13 of the nonwoven fibrous material 4. The plasma treatment provides a surface modification to the nonwoven fibers 4a which enhances the surface energy, thereby improving the wettability and adhesion properties of the nonwoven material 4. The plasma treatment thus leads to an adhesion improvement between a tape adhesion zone 12 of a disposal tape 10 and the modified attachment zone 13 of the nonwoven fibrous material 4.

### Example

Measurement of adhesive strength between disposal tape and backsheet material.

A method was used to determine the adhesive strength between the disposable tape and the backsheet nonwoven material of a diaper when subjected to a defined pulling speed in a tensile tester. The test was conducted at least 24 hours after the product had been manufactured and packed in a bag. An Instron tensile tester with a 50 or 100 N load cell connected to a computer running suitable software was used for the test. The back-waist part of the diaper was secured in a fixture, specially designed for the method, in the Instron tensile tester. The tensile tester pulled the tape at 300 mm/min in a 90°angle and registered the maximum force generated during the test. One test series with disposal tape adhered with glue on a heat-treated zone on the backsheet nonwoven material of the diaper according to the present disclosure and one test series of reference sample glued directly to the backsheet nonwoven material were tested. The result is shown in Table 1. From the result, it is evident with a confidence interval of 95%, that the modified zone of the backsheet nonwoven material according to the present disclosure increased the bonding strength to the disposal tape considerably.

**Table 1**

| Sample No | Description | Result of tensile testing (N) |
|---|---|---|
| 1 | Tape adhered with glue on a heat treated backsheet nonwoven material | 28,885 |
| 2 (reference) | Tape adhered with glue directly on a backsheet nonwoven material | 25,561 |

## Claims

1. A pant-type absorbent article (1) such as a pant diaper, a sanitary pant or incontinence pant, said article (1) comprising an outer cover (2) being disposed on a garment facing side (5) of said absorbent article (1), said article (1) having a front portion (7), a back portion (8) and a crotch portion (9) therebetween, said article (1) comprising an absorbent core (25) arranged in said crotch portion (9) on a wearer facing side of said outer cover (2), said outer cover (2) comprising a nonwoven fibrous material (4) on a garment-facing side (3) thereof and wherein a disposal tape (10) comprising a tape adhesive zone (12) is permanently attached at said tape adhesive zone (12) to said outer cover (2) in an attachment zone (13) on said nonwoven fibrous material (4) of said outer cover (2) in said back portion (8) of said article (1), said disposal tape (10) having a folded pre-disposal configuration and an unfolded disposal configuration, **characterized in that** said outer cover attachment zone (13) is constituted by a modified portion of said nonwoven fibrous material (4) wherein a bond strength between said tape adhesive zone (12) and said modified attachment zone (13) is greater than a bond strength between said tape adhesive zone (12) and a non-modified portion (14) of said nonwoven fibrous material (4), and wherein said modified attachment zone (13) has a modified cross section appearance, such that said nonwoven fibers (4a) are at least partly consolidated and/or flattened within said attachment zone (13).

2. The pant-type absorbent article (1) according to claim 1, **characterized in that** said modified attachment zone (13) is a heat-treated zone, such as a heat-treated zone formed by means of a heat embossing, ultra sound, heated air or IR.

3. The pant-type absorbent article (1) according to any one of claims 1-2, **characterized in that** said modified attachment zone (13) is a compressed area, such as a compressed zone formed by means of embossing.

4. The pant-type absorbent article (1) according to any one of claims 1-3, **characterized in that** said modified attachment zone (13) is a compressed and heat-treated area, such as a heat compressed zone formed by means of heat embossing.

5. The pant-type absorbent article (1) according to any one of the preceding claims, **characterized in that** said modified attachment zone (13) is a plasma-treated zone or a corona-treated zone.

6. The pant-type absorbent article (1) according to any one of the preceding claims, **characterized in that** said outer cover () is a laminate of said nonwoven fibrous material (4) and at least one further material layer, such as a nonwoven material and/or a thermoplastic film.

7. The pant-type absorbent article (1) according to any one of the preceding claims, **characterized in that** said nonwoven fibrous material (4) of said outer cover (2) comprises a plurality of discrete bonds wherein a cumulated surface area of said bonds is in the range of from 1% to 25% of the total surface area of said nonwoven fibrous material (4).

8. The pant-type absorbent article (1) according to any of the preceding claims, **characterized in that** said modified attachment zone (13) has a cumulated bond surface area of at least 30% of the total surface area of said modified attachment zone (13).

9. The pant-type absorbent article (1) according to any one of the preceding claims, **characterized in that** said disposal tape (10) has an outer circumferential edge (15) in said folded pre-disposal configuration and that said outer cover attachment zone (13) has an outer circumferential edge (16), and that said outer cover attachment zone (13) extends to or beyond, such as by at least 1 mm, at least 2 mm, or at least 5 mm, the outer circumferential edge (15) of the disposal tape (10) when arranged in said unfolded pre-disposal configuration.

10. The pant-type absorbent article (1) according to any one of the preceding claims, **characterized in that** said attachment zone (13) is modified in an intermittent pattern or in a continuous pattern covering entirely the outer cover attachment zone (13).

11. The pant-type absorbent article (1) according to any one of the preceding claims, **characterized in that** the disposal tape (10) is rectangular and is z-folded when in said pre-disposal configuration.

12. The pant-type absorbent article (1) according to any one of the preceding claims **characterized in that** the disposal tape (10) comprises or consists of thermoplastic film.

13. The pant-type absorbent article (1) according to any one of the preceding claims, **characterized in that** the disposal tape (10) is permanently attached to the attachment zone (13) by means of an adhesive.

14. A method of permanently attaching a disposal tape (10) to an outer cover (2) of a pant-type absorbent article (1), comprising:
a) modifying a zone (13) on a nonwoven fibrous material (4) with heat treatment, plasma treatment, by compressing said zone or a combination of two or more of said treatments to provide a modified attachment zone (13);
b) permanently attaching a disposal tape (10) comprising a tape adhesive zone (12) to said nonwoven fibrous material (4) by adhesively bonding said tape adhesive zone (12) to said modified attachment zone (13);
c) producing a pant-type absorbent article (1) comprising said nonwoven fibrous material (4) as an outer cover (2) being disposed on a garment facing side (5) of said absorbent article (1), said article (1) having a front portion (7), a back portion (8) and a crotch portion (9) therebetween and comprises an absorbent core (25) arranged in said crotch portion (9),
wherein said zone modification step a) and said disposal tape attachment step b) are carried out prior to, during or after said pant-type absorbent article assembly step c).

15. The method of permanently attaching a disposal tape (10) to an outer cover (2) of a pant-type absorbent article (1) according to claim 16 or 17, wherein said step c) of producing an absorbent article (1) includes elastifying said outer cover (2) by means of elastic elongated elements or an elastic material or wherein said nonwoven fibrous material (4) is an elastic nonwoven fibrous material, providing an at least partly elastic outer cover (2) and wherein said at least partly elastic outer cover (2) is kept in a stretched state during said zone modification step a) and said disposal tape (10) attachment step b).

## Patentansprüche

1. Hosenähnlicher, saugfähiger Artikel (1), wie z. B. eine Hosenwindel, eine Hygienehose oder Inkontinenzhose, wobei der Artikel (1) eine Außenhülle (2) umfasst, die an einer zur Bekleidung gerichteten Seite (5) des saugfähigen Artikels (1) angeordnet ist, wobei der Artikel (1) einen vorderen Teil (7), einen hinteren Teil (8) und einen Schrittteil (9) dazwischen aufweist, wobei der Artikel (1) einen saugfähigen Kern (25) umfasst, der in dem Schrittteil (9) an einer zum Träger gerichteten Seite der Außenhülle (2) angeordnet ist, wobei die Außenhülle (2) einen Faservliesstoff (4) an einer zur Bekleidung gerichteten Seite (3) davon umfasst und wobei ein abnehmbarer Klebestreifen (10), umfassend eine Klebebandzone (12), permanent an der Klebebandzone (12) an der Außenhülle (2) in einer Befestigungszone (13) an dem Faservliesstoff (4) der Außenhülle (2) an dem hinteren Teil (8) des Artikels (1) befestigt ist, wobei der abnehmbare Klebestreifen (10) eine gefaltete Vorabnahmekonfiguration und eine ungefaltete Abnahmekonfiguration aufweist, **dadurch gekennzeichnet, dass** die Befestigungszone (13) der Außenhülle aus einem modifizierten Teil des Faservliesstoffs (4) besteht, wobei eine Klebefestigkeit zwischen der Klebebandzone (12) und der modifizierten Befestigungszone (13) größer ist als eine Klebefestigkeit zwischen der Klebebandzone (12) und einem nicht modifizierten Teil (14) des Faservliesstoffs (4) und wobei die modifizierte Befestigungszone (13) eine modifizierte Querschnittserscheinung aufweist, sodass die Faservliese (4a) mindestens teilweise innerhalb der Befestigungszone (13) konsolidiert und/oder flachgedrückt sind.

2. Hosenähnlicher, saugfähiger Artikel (1) nach Anspruch 1, **dadurch gekennzeichnet, dass** die modifizierte Befestigungszone (13) eine wärmebehandelte Zone ist, wie z. B. eine wärmebehandelte Zone, die durch eine Wärmeprägung, Ultraschall, erwärmte Luft oder IR gebildet ist.

3. Hosenähnlicher, saugfähiger Artikel (1) nach einem der Ansprüche 1-2, **dadurch gekennzeichnet, dass** die modifizierte Befestigungszone (13) ein verdichteter Bereich ist, wie z. B. eine verdichtete Zone, die durch Prägen gebildet ist.

4. Hosenähnlicher, saugfähiger Artikel (1) nach einem der Ansprüche 1-3, **dadurch gekennzeichnet, dass** die modifizierte Befestigungszone (13) ein verdichteter und wärmebehandelter Bereich ist, wie z. B. eine wärmeverdichtete Zone, die durch Wärmeprägung gebildet ist.

5. Hosenähnlicher, saugfähiger Artikel (1) nach einem der vorstehenden Ansprüche, **dadurch gekennzeichnet, dass** die modifizierte Befestigungszone (13) eine plasmabehandelte Zone oder eine koronabehandelte Zone ist.

6. Hosenähnlicher, saugfähiger Artikel (1) nach einem der vorstehenden Ansprüche, **dadurch gekennzeichnet, dass** die Außenhülle () aus einem Laminat von dem Faservliesstoff (4) und mindestens einer weiteren Materialschicht, wie z. B. einem Vliesstoff und/oder einer thermoplastischen Folie, besteht.

7. Hosenähnlicher, saugfähiger Artikel (1) nach einem der vorstehenden Ansprüche, **dadurch gekennzeichnet, dass** der Faservliesstoff (4) der Außenhülle (2) eine Vielzahl von einzelnen Klebeverbindungen umfasst, wobei ein kumulierter Oberflächenbereich der Klebeverbindungen im Bereich von 1 % bis 25 % des gesamten Oberflächenbereichs des Faservliesstoffs (4) liegt.

8. Hosenähnlicher, saugfähiger Artikel (1) nach einem der vorstehenden Ansprüche, **dadurch gekennzeichnet, dass** die modifizierte Befestigungszone (13) einen kumulierten Klebeverbindungs-Oberflächenbereich von mindestens 30% des gesamten Oberflächenbereichs der modifizierten Befestigungszone (13) aufweist.

9. Hosenähnlicher, saugfähiger Artikel (1) nach einem der vorstehenden Ansprüche, **dadurch gekennzeichnet, dass** der abnehmbare Klebestreifen (10) eine äußere, umfangsseitige Kante (15) in der gefalteten Vorabnahmekonfiguration aufweist und dass die Befestigungszone (13) der Außenhülle eine äußere, umfangsseitige Kante (16) aufweist und dass sich diese Befestigungszone (13) der Außenhülle bis zu der äußeren, umfangsseitigen Kante (15) des abnehmbaren Klebestreifens (10) oder darüber hinaus, wie z. B. um mindestens 1 mm, mindestens 2 mm oder mindestens 5 mm, erstreckt, wenn diese in der ungefalteten Vorabnahmekonfiguration angeordnet ist.

10. Hosenähnlicher, saugfähiger Artikel (1) nach einem der vorstehenden Ansprüche, **dadurch gekennzeichnet, dass** die Befestigungszone (13) in einem unterbrochenen Muster oder in einem kontinuierlichen Muster modifiziert ist, welches die gesamte Befestigungszone (13) der Außenhülle bedeckt.

11. Hosenähnlicher, saugfähiger Artikel (1) nach einem der vorstehenden Ansprüche, **dadurch gekennzeichnet, dass** der abnehmbare Klebestreifen (10) rechtwinklig und z-gefaltet ist, wenn sich dieser in der Vorabnahmekonfiguration befindet.

12. Hosenähnlicher, saugfähiger Artikel (1) nach einem der vorstehenden Ansprüche, **dadurch gekennzeichnet, dass** der abnehmbare Klebestreifen (10) eine thermoplastische Folie umfasst oder aus dieser besteht.

13. Hosenähnlicher, saugfähiger Artikel (1) nach einem der vorstehenden Ansprüche, **dadurch gekennzeichnet, dass** der abnehmbare Klebestreifen (10) durch einen Klebstoff permanent an der Befestigungszone (13) befestigt ist.

14. Verfahren zum permanenten Befestigen eines abnehmbaren Klebestreifens (10) an einer Außenhülle (2) eines hosenähnlichen, saugfähigen Artikels (1), umfassend:
a) Modifizieren einer Zone (13) an einem Faservliesstoff (4) mit Wärmebehandlung, Plasmabehandlung, durch Verdichten der Zone oder einer Kombination aus zwei oder mehr der Behandlungen, um eine modifizierte Befestigungszone (13) bereitzustellen;
b) permanentes Befestigen eines abnehmbaren Klebestreifens (10), umfassend eine Klebebandzone (12), an den Faservliesstoff (4) durch eine Klebstoffverbindung der Klebebandzone (12) mit der modifizierten Befestigungszone (13);
c) Produzieren eines hosenähnlichen, saugfähigen Artikels (1), umfassend den Faservliesstoff (4) als eine Außenhülle (2), die an einer zur Bekleidung gerichteten Seite (5) des saugfähigen Artikels (1) angeordnet ist, wobei der Artikel (1) einen vorderen Teil (7), einen hinteren Teil (8) und einen Schrittteil (9) dazwischen aufweist und einen saugfähigen Kern (25) umfasst, der in dem Schrittteil (9) angeordnet ist,
wobei der Zonenmodifizierungsschritt a) und der Befestigungsschritt des abnehmbaren Klebestreifens b) vor, während oder nach dem Zusammenfügungsschritt c) des hosenähnlichen, saugfähigen Artikels durchgeführt werden.

15. Verfahren zum permanenten Befestigen eines abnehmbaren Klebestreifens (10) an einer Außenhülle (2) eines hosenähnlichen, saugfähigen Artikels (1) nach Anspruch 16 oder 17, wobei Schritt c) des Produzierens eines saugfähigen Artikels (1) Elastifizieren der Außenhülle (2) durch elastisch verlängerte Elemente oder ein elastisches Material beinhaltet, oder wobei der Faservliesstoff (4) ein elastischer Faservliesstoff ist, der eine mindestens teilweise elastische Außenhülle (2) bereitstellt und wobei die mindestens teilweise elastische Außenhülle (2) während dem Zonenmodifizierungsschritt a) und dem Befestigungsschritt b) des abnehmbaren Klebestreifens (10) in einem gedehnten Zustand gehalten wird.

## Revendications

1. Article absorbant de type culotte (1) tel qu'une couche-culotte, une culotte hygiénique ou une culotte d'incontinence, ledit article (1) comprenant une enveloppe extérieure (2) disposée sur un côté faisant face au vêtement (5) dudit article absorbant (1), ledit article (1) ayant une partie avant (7), une partie arrière (8) et une partie d'entrejambe (9) entre elles, ledit article (1) comprenant un noyau absorbant (25) disposé dans ladite partie d'entrejambe (9) sur un côté faisant face au porteur de ladite enveloppe extérieure (2), ladite enveloppe extérieure (2) comprenant un matériau fibreux non tissé (4) sur un côté de celle-ci faisant face au vêtement (3) et dans lequel un ruban de mise au rebut (10) comprenant une zone adhésive de ruban (12) est fixé de manière permanente au niveau de ladite zone adhésive de ruban (12) à ladite enveloppe extérieure (2) dans une zone de fixation (13) sur ledit matériau fibreux non tissé (4) de ladite enveloppe extérieure (2) dans ladite partie arrière (8) dudit article (1), ledit ruban de mise au rebut (10) ayant une configuration pliée de pré-mise au rebut et une configuration dépliée de mise au rebut, **caractérisé en ce que** ladite zone de fixation (13) de la couverture extérieure est constituée par une partie modifiée dudit matériau fibreux non tissé (4), dans laquelle une résistance de liaison entre ladite zone d'adhésif de ruban (12) et ladite zone de fixation modifiée (13) est supérieure à une résistance de liaison entre ladite zone d'adhésif de ruban (12) et une partie non modifiée (14) dudit matériau fibreux non tissé (4), et dans lequel ladite zone de fixation modifiée (13) a un aspect de section transversale modifié, de sorte que lesdites fibres non tissées (4a) sont au moins partiellement consolidées et/ou aplaties dans ladite zone de fixation (13).

2. Article absorbant de type culotte (1) selon la revendication 1, **caractérisé en ce que** ladite zone de fixation modifiée (13) est une zone traitée thermiquement, telle qu'une zone traitée thermiquement formée au moyen d'un gaufrage thermique, d'ultrasons, d'air chauffé ou d'IR.

3. Article absorbant de type culotte (1) selon l'une quelconque des revendications 1 à 2, **caractérisé en ce que** ladite zone de fixation modifiée (13) est une zone comprimée, telle qu'une zone comprimée formée au moyen d'un gaufrage.

4. Article absorbant de type culotte (1) selon l'une quelconque des revendications 1 à 3, **caractérisé en ce que** ladite zone de fixation modifiée (13) est une zone comprimée et traitée thermiquement, telle qu'une zone comprimée thermiquement formée au moyen d'un gaufrage à chaud.

5. Article absorbant de type culotte (1) selon l'une quelconque des revendications précédentes, **caractérisé en ce que** ladite zone de fixation modifiée (13) est une zone traitée par plasma ou une zone traitée par corona.

6. Article absorbant de type culotte (1) selon l'une quelconque des revendications précédentes, **caractérisé en ce que** ladite enveloppe extérieure est un stratifié dudit matériau fibreux non tissé (4) et d'au moins une autre couche de matériau, tel qu'un matériau non tissé et/ou un film thermoplastique.

7. Article absorbant de type culotte (1) selon l'une quelconque des revendications précédentes, **caractérisé en ce que** ledit matériau fibreux non tissé (4) de ladite enveloppe extérieure (2) comprend une pluralité de liaisons discrètes, dans lequel une aire de surface cumulée desdites liaisons est dans la gamme de 1 % à 25 % de l'aire de surface totale dudit matériau fibreux non tissé (4).

8. Article absorbant de type culotte (1) selon l'une quelconque des revendications précédentes, **caractérisé en ce que** ladite zone de fixation modifiée (13) présente une surface de liaison cumulée d'au moins 30% de la surface totale de ladite zone de fixation modifiée (13).

9. Article absorbant de type culotte (1) selon l'une quelconque des revendications précédentes, **caractérisé en ce que** ledit ruban de mise au rebut (10) a un bord circonférentiel extérieur (15) dans ladite configuration pliée de pré-mise au rebut et **en ce que** ladite zone de fixation (13) de l'enveloppe extérieure a un bord circonférentiel extérieur (16), et **en ce que** ladite zone de fixation (13) de l'enveloppe extérieure s'étend vers ou au-delà, par exemple d'au moins 1 mm, d'au moins 2 mm ou d'au moins 5 mm, du bord circonférentiel extérieur (15) du ruban de mise au rebut (10) lorsqu'il est disposé dans ladite configuration dépliée de pré-mise au rebut.

10. Article absorbant de type culotte (1) selon l'une quelconque des revendications précédentes, **caractérisé en ce que** ladite zone de fixation (13) est modifiée selon un motif intermittent ou selon un motif continu couvrant entièrement la zone de fixation (13) de l'enveloppe extérieure.

11. Article absorbant de type culotte (1) selon l'une quelconque des revendications précédentes, **caractérisé en ce que** le ruban de mise au rebut (10) est rectangulaire et est plié en z lorsqu'il est dans ladite configuration de pré-mise au rebut.

12. Article absorbant de type culotte (1) selon l'une quelconque des revendications précédentes, **caractérisé en ce que** le ruban de mise au rebut (10) comprend ou consiste en un film thermoplastique.

13. Article absorbant de type culotte (1) selon l'une quelconque des revendications précédentes, **caractérisé en ce que** le ruban de mise au rebut (10) est fixé de manière permanente à la zone de fixation (13) au moyen d'un adhésif.

14. Procédé de fixation permanente d'un ruban de mise au rebut (10) à une enveloppe extérieure (2) d'un article absorbant de type culotte (1), comprenant :
a) la modification d'une zone (13) sur un matériau fibreux non tissé (4) par traitement thermique, traitement au plasma, par compression de ladite zone ou par une combinaison de deux ou plusieurs desdits traitements pour fournir une zone de fixation modifiée (13) ;
b) la fixation permanente d'un ruban de mise au rebut (10) comprenant une zone adhésive de ruban (12) audit matériau fibreux non tissé (4) en liant de manière adhésive ladite zone adhésive de ruban (12) à ladite zone de fixation modifiée (13) ;
c) produire un article absorbant de type culotte (1) comprenant ledit matériau fibreux non tissé (4) en tant qu'enveloppe extérieure (2) disposée sur un côté faisant face au vêtement (5) dudit article absorbant (1), ledit article (1) ayant une partie avant (7), une partie arrière (8) et une partie d'entrejambe (9) entre elles et comprend un noyau absorbant (25) disposé dans ladite partie d'entrejambe (9),
dans lequel ladite étape de modification d'une zone a) et ladite étape de fixation d'un ruban de mise au rebut b) sont effectuées avant, pendant ou après ladite étape d'assemblage d'article absorbant de type culotte c).

15. Procédé de fixation permanente d'un ruban de mise au rebut (10) à une enveloppe extérieure (2) d'un article absorbant de type culotte (1) selon la revendication 16 ou 17, dans lequel ladite étape c) de production d'un article absorbant (1) comprend l'élastification de ladite enveloppe extérieure (2) au moyen d'éléments élastiques allongés ou d'un matériau élastique ou dans lequel ledit matériau fibreux non tissé (4) est un matériau fibreux non tissé élastique, la fourniture d'une enveloppe extérieure (2) au moins partiellement élastique et dans lequel ladite enveloppe extérieure (2) au moins partiellement élastique est maintenue dans un état étiré pendant ladite étape de modification d'une zone a) et ladite étape de fixation d'un ruban de mise au rebut b).
